(19) Europäisches Patentamt
European Patent Office
Office européen des brevets

(11) **EP 0 942 654 B1**

(12) **EUROPÄISCHE PATENTSCHRIFT**

(45) Veröffentlichungstag und Bekanntmachung des Hinweises auf die Patenterteilung:
**17.07.2002 Patentblatt 2002/29**

(21) Anmeldenummer: **97951896.6**

(22) Anmeldetag: **13.11.1997**

(51) Int Cl.$^7$: **A21D 8/04**

(86) Internationale Anmeldenummer:
**PCT/EP97/06331**

(87) Internationale Veröffentlichungsnummer:
**WO 98/23162 (04.06.1998 Gazette 1998/22)**

(54) **VERFAHREN ZUR HERSTELLUNG VON FRISCHHALTE-BACKWAREN**

METHOD FOR PRODUCTION OF STAY-FRESH BAKED GOODS

PROCEDE POUR PREPARER DES ARTICLES DE BOULANGERIE "FRAICHEUR"

(84) Benannte Vertragsstaaten:
**AT BE CH DE DK ES FI FR GB IT LI NL SE**

(30) Priorität: **22.11.1996 DE 19648343**

(43) Veröffentlichungstag der Anmeldung:
**22.09.1999 Patentblatt 1999/38**

(73) Patentinhaber: **AB Enzymes GmbH**
**64293 Darmstadt (DE)**

(72) Erfinder:
• **SCHUSTER, Erwin**
**D-64625 Bensheim (DE)**
• **SPRÖSSLER, Bruno**
**D-64380 Ro dorf (DE)**
• **HOFEMEISTER, Jürgen**
**D-06466 Gatersleben (DE)**

(74) Vertreter:
**Weisert, Annekäte, Dipl.-Ing. Dr.-Ing. et al**
**Patentanwälte**
**Kraus & Weisert**
**Thomas-Wimmer-Ring 15**
**80539 München (DE)**

(56) Entgegenhaltungen:
**DD-A- 225 711        DD-A- 288 395**

• **HOFEMEISTER, B. ET AL.: "The gene amyE (TV1) codes for a nonglucogenic alpha-amylase from Thermoactinomyces vulgaris 94-2A in Bacillus subtilis." APPLIED AND ENVIRONMENTAL MICROBIOLOGY, Bd. 60, Nr. 9, September 1994, Seiten 3381-3389, XP002068742 in der Anmeldung erwähnt**
• **TÄUFEL, A. ET AL.: "Einfluss der alpha-amylase auf die Proteasewirkung in kleberhaltigen Substraten." DIE NÄHRUNG, Bd. 22, Nr. 9, 1978, DE, Seiten 819-826, XP002068743**

**Beschreibung**

Gebiet der Erfindung

Die Erfindung betrifft ein Verfahren zur Herstellung von Backwaren aus Getreideprodukten unter Verwendung von Enzymen, mit dem Ziel, das Altbackenwerden der Backwaren zu verhindern.

Stand der Technik

Die Verhinderung des Altbackenwerdens oder, positiv ausgedrückt: die Sicherung der Frischhaltung von Backwaren ist ein Problem seit Brot gebacken wird. Bislang haben sich zwar die Vertriebsstellen für Backwaren, die Bäckereien und auch die Verbraucher damit abgefunden und darauf eingestellt, daß z.B. Brötchen am gleichen Tag verkauft und gegessen werden müssen. Eine Verlängerung der Frischhaltung um mehrere Tage wäre erstrebenswert und würde das Konsumverhalten und die Verteilungsstrategie für dieses wichtige Grundnahrungsmittel entscheidend ändern.

Im Zuge der Innovationsoffensive der Lebensmitteltechnologie in den letzten Jahren hat sich die Enzymtechnologie dieses Problems angenommen und bereits eine Reihe von Lösungen vorgeschlagen.

Der Vorgang des Altbackenwerdens ist sehr komplex und wird keinesfalls vollständig verstanden. Auch das Erscheinungsbild des Effektes ist mehrschichtig. Es werden folgende störende Phänomene beobachtet:

1. Eine Zunahme der Festigkeit der Krume. Das Brot wird hart.
2. Die Brotkruste wird ledrig oder gummiartig.
3. Verlust an Brotaroma. Sogar eine negative Geruchsnote kann auftreten.

Die Fachwelt ist sich einig, daß das Altbackenwerden der Backwaren mit der Retrogradation der Stärke und mit der damit verbundenen Änderung des Wasserhaltevermögens zusammenhängt. Stärke ist ein wesentlicher Inhaltsstoff von Backwaren, sie liegt im Teig in Form von mit Protein umhüllten Körnern vor. Durch den Backvorgang verkleistert die Stärke und nimmt viel Wasser auf, während das Protein koaguliert. Unmittelbar nach dem Backen beginnt die Stärke unter Wasserabgabe zu rekristallisieren (= zu retrogradieren). Die Festigkeit der Krume nimmt zu, was in den ersten vier Stunden noch als Vorteil empfunden wird. Die Schnittfähigkeit und die Kaueigenschaften verbessern sich vorerst. Man nimmt an, daß zuerst die unverzweigte Stärke-Fraktion, also die Amylose kristallisiert, bei weiterer Lagerung auch die verzweigte Fraktion der Stärke, das Amylopektin. Die Krume versteift sich dabei, wird im Laufe der Zeit immer zähelastischer, später trockener und härter: das Brot ist altbacken geworden.

Die Kruste dagegen verliert beim Lagern die Rösche. Man nimmt an, daß durch die Rekristallisation Wasser frei wird, dieses aus der Krume nach außen diffundiert und die Kruste durchfeuchtet. Daher wird die Kruste zäh und ledrig.

Der Aromaverlust des altbackenen Brotes könnte unter anderem durch Einschluß der Aromastoffe in die Stärkehelix erklärt werden.

Es ist unbestritten, daß die ursächliche Schlüsselreaktion für alle diese Erscheinungen des Altbackenwerdens die Stärkeretrogradierung ist. Diese zu unterdrücken oder zu umgehen ist Gegenstand einer Reihe von Schutzrechten und Veröffentlichungen.

Eine Strategie, die starke Verfestigung der Krume bei Lagerung wenigstens teilweise zu verhindern, ist schon lange bekannt: Man stellt die Krume von vornherein weicher ein. Mittel der Wahl sind Emulgatoren, wie Lecithin, Lysolecithin, Diacetylweinsäureester oder Mono- und Diglyceridester, die dem Teig zugesetzt werden und von Anfang an eine besonders weiche Krumenstruktur erzeugen. Es-wird auch postuliert, daß die Mono- und Diglyceridester einerseits das bei der Rekristallisation freiwerdende Wasser aufnehmen, andererseits durch Assoziation an der Amylose deren Rekristallisation soweit stören, daß sie nicht mehr vollständig ablaufen kann.

Auch der Einsatz von Pilz-alpha-Amylase, z.B. aus Aspergillus oryzae, zielt in diese Richtung. Sie greift geschädigte Stärkekörner an, erniedrigt dadurch die Viskosität des Teiges und stellt fermentierbare Zucker bereit. Die Folge ist ein hohes Volumen der fertigen Backware, das mit einer weicheren Krume einhergeht: Eine besonders weiche Krume läßt den Vorgang des Verfestigens beim Altern nicht so deutlich hervortreten.

Abgesehen davon, daß das frische Brot zu weich ist, verhindert diese Strategie nicht oder zu wenig die Entwicklung einer gummiähnlichen Konsistenz der Krume, sowie die anderen Brotfehler des Altbackenwerdens.

Daher ist eine weitere Strategie, nämlich die Verhinderung der Retrogradierung durch eine teilweise enzymatische Hydrolyse der beiden Stärkefraktionen erfolgversprechender. Man nimmt an, daß die bei der Hydrolyse- der Stärke entstehenden Bruchstücke zu kurz sind um rekristallisieren zu können. Die Bruchstücke assoziieren an die verbliebene hochmolekulare Stärke und verhindern weitgehend auch deren Rekristallisation.

Eine enzymatische Hydrolyse der Stärke soll nach Ansicht der Fachwelt möglichst bei der Verkleisterungstemperatur, also oberhalb von etwa 70°C stattfinden. Diese Temperaturen werden im Backpozeß ohne weiteres erreicht und überschritten. Das Dilemma der enzymatischen Behandlung ist indes, daß nur eine teilweise Hydrolyse stattfinden darf: nicht zu gering und nicht zu hoch. Ein zu geringer Hydrolysegrad führt nicht zu einem Frischhalteeffekt. Er ist gegeben, wenn man z.B. stärkespaltende Enzyme mit zu niedriger Thermostabilität, wie die oben erwähnte Pilz-alpha-Amylase einsetzt. Hier hat das Enzym bereits seine Aktivität eingebüßt, wenn im Zuge der Temperaturbelastung des Backprozesses die Verkleisterung anfängt, sodaß die Hydrolyse der Stärke zu gering ist.

Die Verwendung von Bakterien-alpha-Amylasen, z.B. aus Bacillus subtilis oder Bacillus stearothermophilus führt zu zu hohen Hydrolysegraden. Sie sind sehr thermostabil und werden während des Backprozesses kaum desaktiviert, sie wirken sogar in der Abkühlphase nach. Die Folge ist ein zu weitgehender Abbau der Stärke, der sich in einer feuchten, klebrigen Krume äußert. Durch eine genaue Dosierung dieses Enzyms allein läßt sich die gewünschte Teilhydrolyse der Stärke schwer in den Griff bekommen.

Es stellt sich somit die Aufgabe, die Stärke nur bis zu einem bestimmten Hydrolysegrad abzubauen, mit der Maßgabe einer ausreichenden Dosierungstoleranz für die Enzymzugabe.

In US 4 416 903 wird vorgeschlagen, die bekannte Pilz-alpha-Amylase durch Einbettung in ein gegen thermische Belastung stabilisierendes Zuckermedium für die Zwecke der Frischhaltung zu verwendbar zu machen. Das Verfahren kommt nicht ohne Emulgator- und Zuckerzsatz aus und kann für eine Reihe von Backwaren, wo Zuckerzusatz unerwünscht ist, nicht eingesetzt werden.

Die PCT Anmeldung WO 89/08403 betrifft ebenfalls den Einsatz von Pilz-alpha-Amylase in der Brotfrischhaltung. Hier wird vorgeschlagen, eine säurestabile Pilz-alpha-Amylase zu verwenden, die im leicht sauren Medium des Teiges auch bei den erhöhten Temperaturen des Backprozesses noch ausreichend Wirkung zeigt, d.h. Stärke bis zu einem bestimmten Hydrolysegrad spaltet. Das Enzym wird aus schwarzsporenden Aspergillusstämmen isoliert und hat ein pH-Optimum von 3 bis 5 bei 60 bis 70 °C.

Generell muß bei den oben beschriebenen Verwendungen der Pilz-alpha-Amylasen mit relativ hohen Enzymdosagen gearbeitet werden, um einen Effekt zu erzielen.

In DD 225 711 wird eine Thermitase mit alpha-amylolytischer Aktivitat durch Thermactinomyces vulgaris hergestellt. Die Enzymaktivitat wird untersucht.

In US 4 654 216 wird versucht, das Problem der Brotfrischhaltung durch eine spezielle Spezifität des verwendeten Enzyms zu lösen. Es wird - zusammen mit Bakterienamylase oder Malzamylase - eine Pullulanase eingesetzt. Sie soll vorwiegend das verzweigte Amylopectin spalten und so die Wirkung der Amylasen unterstützen. Da die verwendeten Enzyme thermostabil sind, besteht hier weiterhin das Problem einer punktgenauen Enzymdosierung, die in der Praxis zu Schwierigkeiten führen muß.

Ein Lösungsweg, der ebenfalls über die Spezifität des verwendeten Enzyms läuft, wird in der PCT-Anmeldung WO 91/04669 beansprucht. Hier werden maltogene Exoamylasen, auch beta-Amylasen genannt, für die Verhinderung des Altbackenwerdens vorgeschlagen. Diese Enzymspezies spaltet aus Stärkemolekülen ausschließlich Maltose ab. Es werden zwei verschiedene beta-Amylasen aus Bacillus angeführt. Deren Quelle und Herstellung sind in EP 234 858 und US 4 604 355 beschrieben. Da diese Enzyme sehr thermostabil sind, - beide Enzyme behalten nach einer 30 minütigen thermischen Belastung von 80°C noch über 50 % ihrer Aktivität - ist anzunehmen ,daß ihre Wirkung zeitlich nicht begrenzt ist, daß sie also während des gesamten Backprozesses und darüber hinaus wirken. Die Enzyme dürften so zumindest zum Teil den Backprozeß überleben und dadurch in aktivem Zustand auch noch in der fertigen Backware enthalten sein. In der Lebensmitteltechnologie legt man Wert darauf, daß Enzyme im verzehrbereiten Produkt in inaktiviertem Zustand vorliegen, was hier aber nicht sicher gegeben und von Nachteil ist. Die Wirkung dieser Enzyme dürfte in einer quantitativ begrenzten Spaltung in die niedermolekularen Bruchstücke bestehen, die das Wasserhaltevermögen verbessern und gleichzeitig die Rekristallisation der Reststärke verhindern. Die Reaktion läuft bis zum beta-Grenzdextrin und bleibt dort stehen. Nachteilig ist auch, daß die Spaltprodukte ausschließlich Zucker sind, was fallweise aus Geschmacksgründen unerwünscht ist.

Auch in EP 412 607 werden zur Verhinderung des Altbackenwerdens thermostabile Exoglucanasen wie beta-Amylasen oder Amyloglucosidasen vorgeschlagen. Sie können durch alpha-1,6-Endoglucanasen wie z.B. Pullulanase ergänzt oder ersetzt werden. Auch hier sind die Enzyme während des Backens zwischen 80 und 95°C wirksam, und ihre vollständige Inaktivierung am Ende des Backprozesses ist nicht sicher. Durch die Wahl dieser Spezifitäten soll vor allem die Amylopektinkomponente selektiv hydrolysiert werden, wobei die Reaktion auch über das Grenzdextrin hinaus gehen kann. Damit erhebt sich auch hier das Problem einer punktgenauen Dosierung des Enzyms.

Aufgabe und Lösung

Die Aufgabe zur Lösung des Brotfrischhalteproblems besteht somit in einer gezielten enzymatischen Teilhydrolyse der Stärke. Folgende Forderungen sollen zusätzlich erfüllt werden:

   1. Zur Erzielung des Effekts sollen keine hohen Enzymdosagen notwendig sein

2. Die Enzymzugabe soll dosierungstolerant sein

3. Nach dem Backprozeß soll das Enzym vollständig desaktiviert sein

4. Die Spaltprodukte sollen eine möglichst geringe Geschmacksveränderung bewirken, insbesondere sollen sie nicht ausschließlich aus Zucker bestehen.

5. Das Brotaroma soll möglichst erhalten bleiben.

Die Aufgabe wird durch Verwendung einer alpha-Amylase, die sich in Herkunft, Spezifität und Thermostabilität vom Stand der Technik unterscheidet, gelöst. Diese alpha-Amylase wird von Thermoactinomyces vulgaris produziert.

Herstellung des Enzyms

Alpha-Amylase aus Thermoactinomyces vulgaris wurde in DD 288 395 offenbart. Es wird hier ihre Herstellung durch Fermentation von Thermoactinomyces vulgaris, sowie ihre Verwendung zur Spaltung von Stärke unter Bildung maltose- und maltotriosereicher Hydrolysate beschrieben.

Das Enzym hat einen isoelektrischen Punkt von 5.57. ein pH-Optimum zwischen 4 und 6 und eine relativ geringe Thermostabilität. So wird nach 20 minütigen thermischen Belastung des Enzyms in wäßriger Lösung bei 70°C keine Aktivität mehr gefunden. Außergewöhnlich ist sein Spaltmuster. Es ist in DD 287 732 beschrieben: Bei der Hydrolyse von nativer Weizenstärke werden lösliche Produkte bestehend aus 4.3 % Glucose, 54.5% Maltose, 20.5% Maltotriose und als Rest lösliche Stärkefragmente erhalten. Somit ist ein Kennzeichen der erfindungsgemäßen alpha-Amylase ein Gehalt von 50 bis 60 Gew.% an Maltose in den löslichen Spaltprodukten bei der Weizenstärke-Hydrolyse.

Das nach den in den obigen DD-Patenten beschriebenen Herstellverfahren der Kultivierung von Thermoactinomyces vulgaris produzierte Enzym kann direkt für die Zwecke der vorliegenden Erfindung, nämlich in dem Verfahren zur Herstellung von Backwaren mit verbesserter Frischhaltung verwendet werden. In Hansen et al., Int. J. Pept. Protein Res. (1994), 44(3), p. 245-252, werden verschiedene Eigenschaften dieses Enzyms beschrieben, wie z.B. die Molmasse und der $K_m$-Wert. Auch die Aminosäuresequenz wird dokumentiert, sowie die Tatsache, daß die in Thermoactinomyces vulgaris enthaltenen Proteinase die alpha-Amylase in zwei Bruchstücke zu spalten vermag, die beide im Sinne der Erfindung aktiv sind.

Es hat sich aber gezeigt, daß Thermoactinomyces vulgaris kein besonders produktiver Stamm zur Erzeugung von alpha-Amylase ist. Daher wurden bereits erfolgreiche Versuche unternommen, dieses Enzym gentechnisch herzustellen. In Arbeiten von z.B. Hofemeister et al., Appl. Environ. Microbiol. (1994), 60(9), p. 3381 - 3389, wird die Isolierung des alpha-Amylase Gens aus Thermoactinomyces vulgaris beschrieben, seine Basensequenz dokumentiert und die Expression des Gens in Escherichia coli und Bacillus subtilis behandelt. B. subtilis ist ein besonders effektiver Wirtsorganismus. Das Thermoactinomyces vulgaris-Gen wurde mit Hilfe eines Genkonstrukts mit einem B. subtilis-Plasmid als Vektor in den Wirtsorganismus eingeschleust. Letzterer wird dann in einem geeigneten Nährmediumt auf Basis Kohlenstoff, Stickstoff und anorganischen Salzen kultiviert. Da die Enzymausbeute beim gentechnischen Verfahren der Herstellung deutlich besser ist, wird dieses bevorzugt.

Anwendung des Enzyms

Im Vergleich zum Stand der Technik war es unerwartet, daß eine alpha-Amylase mit obigen Eigenschaften, nämlich mit dem oben genannten Spaltmuster bei der Stärkehydrolyse, das etwa zwischen dem einer alpha-Amylase und einer beta Amylase liegt, sowie mit einer so geringen Thermostabilität so hervorragende Effekte bei der Brotfrischhaltung bewirkt. Die Wirkung ist unabhängig von der Art der Backwaren,

Unter Backwaren sind in erster Linie solche gemeint, die unter Zusatz von Hefe hergestellt werden. Es sind dies z.B. Weißbrot, Weizen-und Roggen-Mischbrote oder Vollkornbrote.

Das Enzym kann bereits dem Mehl zugemischt werden, das für die Herstellung der Backwaren verwendet wird. Es kann auch im Backmittel enthalten sein, das dem Mehl oder dem Teig zugegeben wird. Häufig mischt man es aber direkt dem Teig zu. Es muß auf jeden Fall im Teig enthalten sein, wenn der Backprozeß beginnt.

Das erfindungsgemäße Frischalteenzym soll in einer Menge zugesetzt werden, die zur Verhinderung des Altbackenwerdens wirksam ist. Die Enzymmenge wird üblicherweise über die Enzymaktivität definiert. Letztere kann z.B. in AZ-Einheiten angegegeben werden. 1 AZ-Einheit entspricht der Enzymaktivität pro Gramm Enzympräparat, welche unter den gegebenen Bedingungen (pH=5, 20 °C, 6%ige Lösung von löslicher Stärke als Substrat) die Spaltung einer 0.75 mMol Oligosaccharid äquivalenten Anzahl glykosidischer Bindungen katalysiert. Siehe auch Willstätter, Waldschmidt-Leitz und Hesse, Z. f. physiol. Chem., Bd. 126, Seite 143, 1922.

Die Analyse wird folgendermaßen durchgeführt:

In eine 50 ml-Weithalsflasche gibt man 5.5 ml 6 %ige Stärkelösung (Merck, Best. Nr. 1252), die durch kurzzeitiges Erhitzen praktisch vollständig gelöst und die mit Natriumacetatpuffer auf pH=5 eingestellt worden war. Sie wird auf 20 °C temperiert. Dann gibt man 2ml der zu bestimmenden Enzymlösung unter Schwenken zu und läßt das Reaktionsgemisch genau 15 min bei 20 °C stehen. Genau zu diesem Zeitpunkt wird die Reaktion mit 0.5 ml 1 n Salzsäure unterbrochen.

Dann gibt man dem Reaktionsansatz 10 ml 0.1 n Jodlösung und sofort 20 ml 0.1 n Natronlaugelösung zu. Nach gründlichem Rühren läßt man den Ansatz 20 min stehen. Anschließend gibt man 5.2 ml 1 n Schwefelsäure zu und titriert mit 0.1 n Natriumthiosulfatlösung auf farblos.

Parallel dazu erstellt man einen Blindwert, indem man die Enzymlösung durch reines Wasser ersetzt.

Die Differenz zwischen Haupt- und Blindwert ergibt den Verbrauch an 0.1 n Jodlösung (V) in ml, der in die Berechnung der AZ- Aktivität folgendermaßen eingeht.

$$AZ = \frac{0.0011288 \times V^2 + 0.23736 \times V}{\text{Einwaage in g}} \times 0.96$$

Die Enzymeinwaage wird so gewählt, daß der Wert für V zwischen 0.8 und 1.5 ml liegt

Je nach Art des Mehls und dem angestrebten Backprodukt werden pro 100 kg Mehl 20 bis 20 000 AZ eingesetzt. Bevorzugt sind 100 bis 10 000 AZ. Besonders bevorzugt sind 300 bis 3000 AZ pro 100 kg Mehl.

Das erfindungsgemäße Frischhalteenzym kann für sich allein als einzige enzymatische Wirksubstanz zugesetzt werden. Selbstverständlich können aber auch andere Enzyme wie z.B. weitere alpha-Amylasen, Glucosidasen, Proteinasen, Lipasen, Lipoxygenasen, Hemicellulasen (Pentosanasen, Xylanasen), Oxidasen oder Transglutaminasen zugesetzt werden. Als besonders wirksam erweist sich in diesem Zusammenhang der Zusatz von backwirksamer Xylanase. Sie erhöht das Backvolumen besonders wirkungsvoll und führt zu einer auffallend weichen Krume. Es wird postuliert, daß dieses Enzym einen Teil der unlöslichen Pentosane wasserlöslich oder zu mindest wasserquellbar macht, diese Inhaltsstoffe somit die Funktion der Wasseraufnahme übernehmen können und dadurch die Frischhaltewirkung des erfindungsgemäßen Enzyms verstärkt wird. Xylanasen werden in Dosagen von 1000 bis 20000 Xylanase-Einheiten pro 100 kg Mehl zugesetzt, vorteilhaft zusammen mit der alpha Amylase aus Thermoactinomyces vulgaris.

Die Aktivitätseinheit für Xylanase ist folgendermaßen definiert: 1 Xylanase-Einheit ist jene Enzymmenge, die in 1 Minute bei 30 °C 1 μmol Xylose aus löslichem Xylan freisetzt. Das Substrat Xylan stammt aus Haferspelzen und wird zur Analyse in 0.25 %iger Lösung bei pH=4.5 eingesetzt. Die Bestimmung der Xylose kann z.B. mit p-Hydroxybenzoesäurehydrazid fotometrisch erfolgen.

Auch auf die anderen üblichen Zusatzstoffe bei der Brotherstellung, wie z.B. an sich bekannte Emulgatoren oder Konservierungsstoffe braucht nicht verzichtet werden.

Vorteilhafte Wirkungen

Bei den nach dem erfindungsgemäßen Verfahren hergestellten Backwaren wird ein vozüglicher Frischhalteeffekt beobachtet. So kann z.B. Weißbrot auch nach vier Tagen Lagerung als frisch bezeichnet werden: Die Krume bleibt weich und saftig, die Kruste wird nicht ledrig. Zur Erzielung des Effekts sind keine hohen Enzymdosagen notwendig. Auch die Dosierungstoleranz ist gut: Selbst bei mehrfacher Überdosierung tritt nicht der von der thermostabilen Bakterien-alpha-Amylase her bekannte Brotfehler einer klebrigen, feuchten Krume auf.

Das erfindungsgemäße Enzym ist nach dem Backprozeß vollständig desaktiviert und kann durch eine Aktivitätsmessung in der fertigen Backware nicht mehr nachgewiesen werden.

Obwohl die im Teig vorhandenen Stärkefraktionen eine Teilhydrolyse erfahren haben, also eine Reihe niedermolekularer, zuckerähnlicher Reaktionsprodukte entstanden sein muß, wird keine Geschmacksveränderung in Form einer Zunahme der Süße beobachtet.

Insgesamt bleiben die Weichheit der Krume, sowie Geschmack und Geruch über längere Lagerung einwandfrei erhalten

Die erfindungsgemääß Thermoactinomyces vulgaris alpha Amylase kann mit den beim Backen üblichen Zusatzstoffen, wie anderen Enzymen und/oder Backemulgatoren, Hydrokolloiden und Konservierungsstoffen vorteilhaft kombiniert werden.

BEISPIELE

Durchführung von Backversuchen

Aus 1500 g Mehl, 870 ml Wasser, 45 g Hefe, 30 g Salz und 5 g Ascorbinsäure wird in einem Spiralkneter (Marke Kemper) ein Teig bereitet. Dabei wird 2 min auf niedriger Stufe 1 und 6 min auf höherer Stufe 2 geknetet. Eine etwaige Enzymzugabe erfolgt zu Beginn des Knetvorganges in die Wasserphase. Die Teigtemperatur beträgt 26-27°C.

Nach einer Teigruhe von 20 min wird der Teig zur Herstellung von Kasten-Weißbrot jeweils in 4 mal 600 g-Stücke geteilt, in die Form eingebracht und 75 min bei 32°C und 80 % relativer Luftfeuchte gegart, anschließend bei 230 °C gebacken. Die Krumenfestigkeit wird am frischen Brot - nachdem es abgekühlt ist, also etwa nach drei Stunden, nach 24 Stunden und nach vier Tagen mit einem Compressimeter bestimmt. Niedrigere Zahlen bedeuten dabei eine weichere Krume und damit eine bessere Frischhaltung.

Beschreibung der Compressimeter-Messung

Es wird ein Compressimeter der Fa. F. Watkins Corporation, West Caldwell, USA, verwendet. Das Gerät mißt die Kompressibilität der Brotkrume. Es zeigt die Kraft in Skalenteilen an, die für das Eindrücken der Krume bis zu einer gegebenen Tiefe notwendig ist.

Eine Brotscheibe von 15 mm Dicke wird in das Gerät eingelegt und dabei unter dem Eindrückstempel zentriert. Mit der rechten Schraube wird die Skala D auf null gestellt. Für frisches Brot stellt man eine Eindringtiefe von 3 mm ein, für altes Brot (Lagerzeit 1 bis 4 Tage) 1.5 mm. Zur Messung wird der Motor angestellt, der mit Hilfe eines Fadenzuges den Stempel eindrückt. Ist die gewünschte Eindringtiefe, die auf Skala D angezeigt wird, erreicht, schaltet man den Motor ab und liest die aufgewendete Kraft auf Skala J ab. Die Skalenteile entsprechen etwa dem Gewicht in Gramm, mit dem der Stempel die Krume eingedrückt hat.

Niedrige Zahlen bedeuten eine weiche Krume und damit auch die bessere Frischhaltung.

Beispiel 1 bis 3

Nach obiger Backvorschrift wird mit folgenden Enzymzusätzen gebacken:

Beispiel 1: ohne Enzymzusatz (=Vergleichsbeispiel)
Beispiel 2: mit 1.3 g Thermoactinomyces vulgaris alpha Amylase (TV-A)//100 kg Mehl mit einer Aktivität von 785 AZ pro Gramm
Beispiel 3 mit 2,6 g Thermoactinomyces vulgaris alpha Amylase (TV-A)//100 kg Mehl mit einer Aktivität von 785 AZ pro Gramm

Die Messung des Frischhalteeffektes wurde mit dem Compressimeter in der oben beschriebenen Weise durchgeführt.

Tabelle 1:

| Compressimeter-Messungen | | | | |
|---|---|---|---|---|
| Beispiel | Zusätze g/100 kg Mehl | Skalenteile | | |
| | | nach 3 Std. | nach 1 Tag | nach 4 Tagen |
| 1 | ohne TV-A | 14 | 27 | 28 |
| 2 | mit 1.3 g TV-A | 10 | 17 | 23 |
| 3 | mit 2.6 g TV-A | 8 | 12 | 16 |

Ergebnis:

Deutlich erkennt man die bessere Kompressibilität und damit die höhere Weichheit der Krume nach Zusatz von Thermoactinomyces vulgaris alpha Amylase selbst nach vier Tagen. Die Einsatzmengen, bezogen auf 100 kg Mehl, betrugen in Beispiel 2...1020 AZ, in Beispiel 3...2040 AZ.

**Patentansprüche**

1. Verfahren zur Herstellung von Backwaren mit verbesserter Frischhaltung, **dadurch gekennzeichnet, dass** dem Mehl, das für die Herstellung der Backwaren verwendet wird oder dem Backmittel, das dem Mehl oder dem Teig zugegeben wird, oder dem Teig vor dem Backprozess alpha-Amylase, die eine geringe Thermostabilität besitzt, bei der Stärkehydrolyse 50 bis 60 Gew.-% an Maltose ergibt, und aus Thermoactinomyces vulgaris erhältlich ist, zugesetzt wird.

**2.** Verfahren nach Anspruch 1, **dadurch gekennzeichnet, dass** die alpha-Amylase gentechnisch produziert wurde.

**3.** Verfahren nach Anspruch 2, **dadurch gekennzeichnet, dass** die alpha-Amylase von einem Wirtsorganismus, der das Gen der alpha-Amylase aus Thermoactinomyces vulgaris enthält, produziert wurde.

**4.** Verfahren nach Anspruch 3, **dadurch gekennzeichnet, dass** der Wirtsorganismus ein Bacillus subtilis-Stamm ist.

**5.** Verfahren nach einem der Ansprüche 1 bis 4, **dadurch gekennzeichnet, dass** dem Teig 20 bis 20 000 AZ-Aktivitätseinheiten des Enzyms bezogen auf 100 kg Mehl zugesetzt werden.

**6.** Verfahren nach einem oder mehreren der Ansprüche 1 bis 5, **dadurch gekennzeichnet, dass** dem Teig zusätzlich ein oder mehrere der an sich bekannten Backenzyme wie Proteinasen, Amylasen, Hemicellulasen, Oxidasen und Transglutaminasen zugesetzt werden.

**7.** Verfahren nach einem oder mehreren der Ansprüche 1 bis 5, **dadurch gekennzeichnet, dass** dem Teig zusätzlich Xylanasen in einer Dosierung von 1000 bis 20 000 Xylanase-Einheiten pro 100 kg Mehl zugesetzt werden.

**8.** Verfahren nach einem oder mehreren der Ansprüche 1 bis 7, **dadurch gekennzeichnet, dass** dem Teig zusätzlich an sich bekannte Backemulgatoren zugesetzt werden.

**9.** Mehl zur Herstellung von Bachwaren mit verbesserter Frischhaltung gemäß Anspruch 1, **dadurch gekennzeichnet, dass** es eine alpha-Amylase, die eine geringe Thermostabilität besitzt, bei der Stärkehydrolyse 50 bis 60 Gew.-% an Maltose ergibt und aus Thermoactinomyces vulgaris erhältlich ist, enthält.

**10.** Mehl nach Anspruch 9, **dadurch gekennzeichnet, dass** die alpha-Amylase gentechnisch produziert wurde.

**11.** Mehl nach Anspruch 10, **dadurch gekennzeichnet, dass** die alpha-Amylase von einem Wirtsorganismus, der das Gen der alpha-Amylase aus Thermoactinomyces vulgaris enthält, produziert wird.

**12.** Mehl nach Anspruch 11, **dadurch gekennzeichnet, dass** der Wirtsorganismus ein Bacillus subtilis-Stamm ist.

**13.** Mehl nach einem der Ansprüche 9 bis 12, **dadurch gekennzeichnet, dass** es 20 bis 20 000 AZ-Aktivitätseinheiten alpha-Amylase auf 100 kg enthält.

**14.** Mehl nach einem oder mehreren der Ansprüche 9 bis 13, **dadurch gekennzeichnet, dass** es zusätzlich ein oder mehrere der an sich bekannten Backenzyme wie Proteinasen, Amylasen, Hemicellulasen, Oxidasen und Transglutaminasen enthält.

**15.** Mehl nach einem oder mehreren der Ansprüche 9 bis 14, **dadurch gekennzeichnet, dass** es zusätzlich Xylanasen in einer Dosierung von 1000 bis 20 000 Xylanase-Einheiten pro 100 kg Mehl enthält.

**16.** Verwendung eines Mehls nach einem der Ansprüche 9 bis 15 zur Herstellung von Backwaren mit verbesserter Frischhaltung.

**Claims**

**1.** A process for the production of baked produce with improved keeping qualities, **characterised in that** alpha-amylase which has low thermostability, wherein starch hydrolysis yields 50 to 60 wt.-% maltose, and which can be obtained from *Thermoactinomyces vulgaris,* is added to the flour which is used for the production of the baked produce, or the baking agent which is added to the flour or the dough, or the dough before the baking process.

**2.** A process according to claim 1, **characterised in that** the alpha-amylase is produced by genetic engineering.

**3.** A process according to claim 2, **characterised in that** the alpha-amylase is produced from a host organism which contains the alpha-amylase gene from *Thermoactinomyces vulgaris.*

**4.** A process according to claim 3, **characterised in that** the host organism is a *Bacillus subtilis* strain.

**5.** A process according to one of claims 1 to 4, **characterised in that** 20 to 20,000 AZ activity units of the enzyme, based on 100 kg flour, are added to the dough.

**6.** A process according to one or more of claims 1 to 5, **characterised in that** one or more *per se* known baking enzymes such as proteinases, amylases, hemicellulases, oxidases and transglutaminases are also added to the dough.

**7.** A process according to one or more of claims 1 to 5, **characterised in that** xylanases in a dosage of 1,000 to 20,000 xylanase units per 100 kg flour are also added to the dough.

**8.** A process according to one or more of claims 1 to 7, **characterised in that** *per se* known baking emulsifiers are also added to the dough.

**9.** Flour for the production of baked produce with improved keeping qualities according to claim 1, **characterised in that** it contains an alpha-amylase which has low thermostability, yields 50 to 60 wt.-% maltose during starch hydrolysis and can be obtained from *Thermoactinomyces vulgaris.*

**10.** Flour according to claim 9, **characterised in that** the alpha-amylase is produced by genetic engineering.

**11.** Flour according to claim 10, **characterised in that** the alpha-amylase is produced from a host organism which contains the alpha-amylase gene from *Thermoactinomyces vulgaris.*

**12.** Flour according to claim 11, **characterised in that** the host organism is a *Bacillus subtilis* strain.

**13.** Flour according to one of claims 9 to 12, **characterised in that** it contains 20 to 20,000 AZ activity units of alpha-amylase per 100 kg.

**14.** Flour according to one or more of claims 9 to 13, **characterised in that** it additionally contains one or more *per se* known baking enzymes such as proteinases, amylases, hemicellulases, oxidases and transglutaminases.

**15.** Flour according to one or more of claims 9 to 14, **characterised in that** it also contains xylanases in a dosage of 1,000 to 20,000 xylanase units per 100 kg flour.

**16.** Use of a flour according to one of claims 9 to 15 for the production of baked produce with improved keeping qualities.

**Revendications**

**1.** Procédé de préparation de produits de boulangerie et de pâtisserie présentant une conservation améliorée, **caractérisé en ce que** l'on ajoute, soit à la farine mise en oeuvre pour la préparation des produits de boulangerie, soit à l'agent de cuisson que l'on mélange à la farine ou à la pâte, ou encore à la pâte avant le procédé de cuisson, une alpha-amylase présentant une faible thermostabilité, produisant, lors de l'hydrolyse de l'amidon, de 50% à 60% en poids de maltose, et que l'on peut obtenir à l'aide de Thermoactinomyces vulgaris.

**2.** Procédé selon la revendication 1, **caractérisé en ce que** l'on a préparé l'alpha-amylase par un procédé de génie génétique.

**3.** Procédé selon la revendication 2, **caractérisé en ce que** l'alpha-amylase a été synthétisée par un organisme hôte qui présente le gène correspondant à l'alpha-amylase et provenant du Thermoactinomyces vulgaris.

**4.** Procédé selon la revendication 3, **caractérisé en ce que**, quant à l'organisme hôte, il s'agit d'une souche de Bacillus subtilis.

**5.** Procédé sèlon l'une quelconque des revendications 1 à 4, **caractérisé en ce que** l'on ajoute l'enzyme à la pâte, à raison de 20 à 20 000 unités d'activité AZ par 100 kg de farine.

**6.** Procédé selon une ou plusieurs des revendications 1 à 5, **caractérisé en ce que** l'on ajoute en plus à la pâte une ou plusieurs enzymes pour la cuisson en boulangerie qui sont généralement connues, telles que les protéinases, les amylases, les hémicellulases, les oxydases et les transglutaminases.

7.  Procédé selon une ou plusieurs des revendications 1 à 5, **caractérisé en ce que** l'on ajoute en plus à la pâte, les xylanases à raison de 1 000 à 20 000 unités de xylanase par 100 kg de farine.

8.  Procédé selon une ou plusieurs des revendications 1 à 7, **caractérisé en ce que** l'on ajoute enplus à la pâte, des émulsifiants pour la cuisson en boulangerie qui sont généralement connus.

9.  Farine pour la préparation de produits de boulangerie et de pâtisserie présentant une conservation améliorée selon la revendication 1, **caractérisée en ce qu'**elle contient une alpha-amylase présentant une faible thermostabilité, produisant, lors de l'hydrolyse de l'amidon, de 50% à 60% en poids de maltose, et que l'on peut obtenir à l'aide du Thermoactinomyces vulgaris.

10. Farine selon la revendication 9, **caractérisée en ce que** l'on a préparé l'alpha-amylase par un procédé de génie génétique.

11. Farine selon la revendication 10, **caractérisée en ce que** l'alpha-amylase est synthétisée par un organisme hôte qui présente le gène correspondant à l'alpha-amylase et provenant du Thermoactinomyces vulgaris.

12. Farine selon la revendication 11, **caractérisée en ce que**, quant à l'organisme hôte, il s'agit d'une souche de Bacillus subtilis.

13. Farine selon l'une quelconque des revendications 9 à 12, **caractérisée en ce qu'**elle contient l'alpha-amylase à raison de 20 à 20 000 unités d'activité AZ par 100 kg de farine.

14. Farine selon une ou plusieurs des revendications 9 à 13, **caractérisée en ce qu'**elle contient en plus une ou plusieurs enzymes pour la cuisson en boulangerie qui sont généralement connues, telles que les protéinases, les amylases, les hémicellulases, les oxydases et les transglutaminases.

15. Farine selon une ou plusieurs des revendications 9 à 14, **caractérisée en ce qu'**elle contient en plus les xylanases à raison de 1 000 à 20 000 unités de xylanase par 100 kg de farine.

16. Mise en oeuvre de la farine selon l'une quelconque des revendications 9 à 15 pour la préparation de produits de boulangerie et de pâtisserie présentant une conservation améliorée.